# EUROPEAN PATENT APPLICATION

(11) **EP 3 238 776 A1**
(43) Date of publication of application: **01.11.2017**
(21) Application number: 17167503.6
(22) Date of filing: 21.04.2017
(51) Int. Cl.: A61N 1/36

(54) **IMPLANTABLE PULSE GENERATOR SYSTEM FOR VAGAL NERVE STIMULATION**

(30) Priority: 26.04.2016 US 201662327455 P
(71) Applicant: BIOTRONIK SE & Co. KG, 12359 Berlin (DE)
(72) Inventor: Lian, Jie, Beaverton, OR 97007 (US); Kraiter, Lauren, Tigard, OR 97223 (US); Kibler, Andrew B., Lake Oswego, OR 97035 (US)
(74) Representative: Keck, Hans-Georg

(57) **Abstract**

The invention refers to an implantable pulse generator system (10) that comprises a nerve stimulation unit (80) for generating and delivery of vagal nerve stimulation pulses, at least one autonomic tone sensor (82) for determining an autonomic status of a user and generating an autonomic status representing signal and a control unit (62) that that is connected to the nerve stimulation unit (80) and the autonomic tone sensor (82). The control unit (62) is configured to control the nerve stimulation unit (80) to generate vagal nerve stimulation pulse trains that are adapted to cause vagal nerve stimulation (VNS) with varying intensity, depending on the autonomic status representing signal. The control unit (62) is further configured to evaluate the autonomic status representing signal in a moving window and to thus generate an evaluated autonomic status representing signal. Further, the control unit (62) is configured to gradually increase the intensity of VNS when the evaluated autonomic status representing signal indicates an autonomic tone that is shifting toward more sympathetic dominance, and gradually decrease the intensity of VNS when the autonomic status representing signal indicates an autonomic tone that is shifting toward more parasympathetic dominance, wherein the gradual increase and the gradual decrease of the intensity of the VNS follow two different curves.

## Description

The application refers to an implantable pulse generator system and a method for autonomic adaptive control of vagal nerve stimulation (VNS).

Vagal nerve stimulation (VNS) systems and methods are inter alia disclosed in US 7,778,703, US 7,783,362, US 8,401,604, US 8,473,068, US 8,712,547, US 2013/0184773 and US 2014/0155949 as well as in Robert J. Henning et al. "Vagal nerve stimulation increases right ventricular contraction and relaxation and heart rate", Cardiovascular Research 32, 1996, 846-853.

Vagal nerve stimulation is applied according to a parameter indicating autonomic tone of the patient, for example the heart rate (HR). Vagal nerve stimulation is equivalent to stimulation of parasympathetic system. Therefore, it is not desired when the heart rate is too low. Common VNS is configured in a way that stimulation stops when the HR sinks below a certain value.

US 6,473,644 B1 discloses methods for adjusting vagal nerve stimulation according to the ventricular heart rate of the patient. Controlling methods for the VNS stimulation frequency according to the heart rate are described. In this context, it is described to use a damped feedback loop with hysteresis, with the heart rate as controlling parameter and VNS stimulation frequency as output.

It is an object of the invention to provide an improved system and method for vagal nerve stimulation.

According to the invention, this object is achieved by means of an implantable pulse generator system that comprises
a nerve stimulation unit for generating and delivery of vagal nerve stimulation pulses,
at least one autonomic tone sensor for determining an autonomic status of a user and
   generating an autonomic status representing signal and
a control unit that that is connected to the nerve stimulation unit and the autonomic tone sensor. The control unit is configured to control the nerve stimulation unit to generate vagal nerve stimulation pulse trains that are adapted to cause vagal nerve stimulation (VNS) with varying intensity, depending on the autonomic status representing signal. The control unit is further configured to evaluate the autonomic status representing signal in a moving window and to thus generate an evaluated autonomic status representing signal. Further, the control unit is configured to gradually increase the intensity of VNS when the evaluated autonomic status representing signal indicates an autonomic tone that is shifting toward more sympathetic dominance, and gradually decrease the intensity of VNS when the autonomic status representing signal indicates an autonomic tone that is shifting toward more parasympathetic dominance, wherein the gradual increase and the gradual decrease of the intensity of the VNS follow two different curves.

Preferably, the autonomic status representing signal is one of or a combination of more than one of the following signals:
- delay from beginning of electrical QRS signal to a vibration pulse measured at the cervical level via an accelerometer attached or adjacent to a VNS cuff.
- QRS duration,
- P wave duration,
- heart sound (contraction-induced pressure waves)
- intra-cardiac pressure.

Accordingly, hysteresis control is introduced to VNS so that the intensity of VNS is modulated not only based on the present autonomic tone, but also based on its past history. Adaptive delay of VNS intensity modulation is further introduced as a means of close loop control of VNS in response to transient change of patient's autonomic tone.

By means of such implantable pulse generating system, an apparatus and method of VNS is provided that ensure physiological adaptation of the VNS therapy to the changing autonomic balance while maintaining maximum appropriate therapy levels.

The invention includes the insight that vagal nerve stimulation (VNS) may be associated with the risk of causing clinically significant cardiac suppression. Thus VNS is preferably activated at higher heart rate and deactivated at lower heart rate. However, simply turning on/off the VNS based on heart rate cutoff values is not optimal since it does not consider the physiological adaptation of the VNS therapy, and may result in withholding therapy unnecessarily. In addition, transient change of heart rate due to natural heart rate variability or the influence of measurement noise in the heart rate sensing unit may cause frequent and unnecessary modulation of VNS therapy.

In a preferred embodiment the autonomic status representing signal represents the instantaneous heart rate on a beat-to-beat basis or the instantaneous atrio-ventricular conduction time and wherein the moving window includes a predetermined number of heart rate values or atrio-ventricular conduction time values, respectively.

In particular, it is preferred if the control unit is further configured to generate the evaluated autonomic status representing signal based on the average of the heart rate values or the atrio-ventricular conduction time values, respectively, in the moving window. Accordingly, the control unit is configured to generate a moving average of the autonomic status representing signal and thus causes a time delay that in turn results in a hysteresis.

It is preferred, if the control unit is further configured to increase the intensity of the VNS in response to a decrease of the heart rate or an increase of the atrio-ventricular conduction time and to decrease the intensity of the VNS in response to an increase of the heart rate or a decrease of the atrio-ventricular conduction time.

In a preferred embodiment, the control unit is further configured to cause an increase or a decrease of the intensity of the VNS only when the autonomic status representing signal or the evaluated autonomic status representing signal is within a predetermined range of values.

Preferably, the implantable pulse generator further comprises an activity sensor for determining an exertion level of a user and generating a metabolic demand-representing signal. The control unit is connected to the activity sensor and the autonomic tone sensor and is configured to control the stimulation unit depending on both, the metabolic demand-representing signal and the autonomic status-representing signal. According to the preferred embodiment, adaptive control of the delivery of vagal nerve stimulation (VNS) is provided by adapting the VNS to both the autonomic status and cardiovascular demand of the patient. The implantable pulse generator comprises at least one activity sensor for measuring the patient's exertion level and at least one autonomic tone sensor for detecting the autonomic status of the patient.

Closed loop control of vagal nerve stimulation (VNS) is desired to maximize the beneficial effect for heart failure treatment while minimizing the risk of severe sympathetic depression. On one hand, it is preferred to activate and/or enhance VNS intensity in order to lower a patient's resting sympathetic tone. On the other hand, it is important to limit and/or inhibit VNS in order to preserve an appropriate degree of sympathetic activation when the patient is engaging in physical or mental activities. However, maintaining the optimal balance between VNS and patient's exertion level remains a technical challenge.

According to one embodiment of the invention, the implantable pulse generator is adapted to be capable of inducing either an increase in vagal tone or an increase in sympathetic tone. Accordingly, an implantable pulse generator system is preferred wherein the control unit is adapted to control the stimulation unit to generate stimulation pulses having stimulation parameters that are configured to induce a VNS effect that is either an increase in vagal tone or an increase in sympathetic tone, depending on both, the cardiovascular demand representing signal and the autonomic status representing signal.

Adaptations to alter the effect of VNS may be stimulation parameters such as amplitude or pulse width, and/or selection of stimulating electrode(s) on the vagal nerve stimulator. Accordingly, an implantable pulse generator system is preferred wherein the control unit and the stimulation unit are adapted to alter the effect of VNS by adjusting stimulation parameters including at least one of amplitude or pulse width, and/or selection of stimulating electrodes.

The response of each electrode or stimulation parameter set can be determined by characterization prior to or during therapy and the result of characterization applied to the automatic adaptive control disclosed herein. Accordingly, it is preferred if the control unit is programmable.

According to a further aspect, if the activity sensor indicates a patient is at rest but the sympathetic tone is elevated, then VNS is activated or its intensity is increased in order to enhance the vagal tone to achieve autonomic balance. According to still a further aspect, if the activity sensor indicates the patient is involved in exertion activities but the sympathetic tone is suppressed, then VNS is adapted to decrease vagal tone or enhance sympathetic tone so that the patient is supported in physical or mental activities without artificially induced limitation on exertion.

Accordingly, it is preferred if the control unit is adapted to control the stimulation unit to generate stimulation pulses having stimulation parameters that are configured to induce an increase in vagal tone if the metabolic demand-representing signal of the activity sensor indicates a low demand and the autonomic status-representing signal of the autonomic tone sensor indicates that the sympathetic tone is elevated.

According to a further aspect, it is preferred if the control unit is adapted to control the stimulation pulse unit to generate stimulation pulses having stimulation parameters that are configured to induce a decrease in vagal tone and/or an increase in sympathetic tone if the metabolic demand-representing signal of the activity sensor indicates an elevated metabolic demand and the autonomic status-representing signal of the autonomic tone sensor indicates that the sympathetic tone is suppressed.

The activity sensor for providing a metabolic demand-representing signal preferably comprises at least one of an accelerometer, a CLS sensor (Closed Loop Stimulation sensor) and/or a minute ventilation sensor. A CLS sensor has been shown to be able to measure both physical and mental exertion of a patient.

The CLS sensor and/or the minute ventilation sensor preferably comprise or are connected to an impedance determination unit that is configured to determine an intracardiac or a transthoracic impedance or both.

By means of such impedance determination unit, it is possible to determine both, an intra-cardiac impedance and a transthoracic impedance. Transthoracic impedance can be evaluated to determine a tidal volume (Minute volume) and a breathing rate (minute rate). Intra-cardiac impedance can be used to determine stroke volume, contractility, heart rate and further metrics of a heart.

The Closed Loop Stimulation sensor is configured to determine a course of intracardiac impedance and to compare the course with a reference course and/or to determine a maximum of the first derivative of the course of intracardiac impedance.

The autonomic tone sensor preferably is configured to process IEGM signals representing an intracardiac electrogram (IEGM). The autonomic tone sensor preferably is adapted to determine one or more of the following metrics from IEGM signals: intrinsic heart rate (HR), intrinsic atrioventricular (AV) conduction time, QRS duration and/or P wave duration.

Alternatively, the autonomic tone sensor preferably is configured to process heart sound signals that reflect contraction-induced pressure waves, intra-cardiac pressure and/or an impedance signal reflecting myocardial contractility.

Thus, the autonomic tone sensor measures a physiological signal that is known to be affected by the autonomic status. For example, the device can use IEGM sensing electrodes to measure the intrinsic heart rate (HR) which is directly modulated by the autonomic status of the patient. Elevated sympathetic tone leads to increase in HR and vice versa. In another example, the intrinsic AV conduction time is known to be affected by the autonomic status of the patient. Elevated sympathetic tone is associated with decrease in intrinsic AV conduction time and vice versa. Another measure of autonomic tone could be achieved by measuring the delay from beginning of electrical QRS signal to a vibration pulse measured at the cervical level via an accelerometer attached or adjacent to a VNS cuff. Such vibration pulse reflects a local change of blood pressure that is caused in a cervical blood vessel by myocardial contraction of the heart. The delay between the QRS signal and the vibration pulse is related to cardiac contractility and current autonomic state relative to a previous autonomic state may be derived. Other physiological parameters can also be measured for the evaluation of autonomic status, including but are not limited to, QRS duration, P wave duration, heart sound (contraction-induced pressure waves), intra-cardiac pressure, etc.

A natural contraction of a heart chamber can be similarly detected by the evaluating electrical signals sensed by the sensing channels. In the sensed electrical signal the depolarization of an atrium muscle tissue is manifested by occurrence of a P-wave. Similarly, the depolarization of ventricular muscle tissue is manifested by the occurrence of a R-wave. The detection of a P-wave or a R-wave signifies the occurrence of intrinsic atrial, As, or ventricular, Vs events, respectively. The AV-delay is the time period between an atrial event and a prescribed point of time of a ventricular event. The heart rate (HR) is inverse to the time period between consecutive ventricular events.

In a healthy heart, initiation of the cardiac cycle normally begins with depolarization of the sinoatrial (SA) node. This specialized structure is located in the upper portion of the right atrium wall and acts as a natural "pacemaker" of the heart. In a normal cardiac cycle and in response to the initiating SA depolarization, the right atrium contracts and forces the blood that has accumulated therein into the ventricle. The natural stimulus causing the right atrium to contract is conducted to right ventricle via the atrioventricular node (AV node) with a short, natural delay, the atrioventricular delay (AV-delay). Thus, a short time after the right atrial contraction (a time sufficient to allow the bulk of the blood in the right atrium to flow through the one-way valve into the right ventricle), the right ventricle contracts, forcing the blood out of the right ventricle to body tissue. A typical time interval between contraction of the right atrium and contraction of the right ventricle might be 60 ms; a typical time interval between contraction of the right ventricle and the next contraction of the right atrium might be 800 ms. Thus, it is an right atrial contraction (A), followed a relatively short time thereafter by a right ventricle contraction (V), followed a relatively long time thereafter by the next right atrial contraction, that produces the desired AV synchrony. Where AV synchrony exists, the heart functions very efficiently as a pump in delivering life-sustaining blood to body tissue; where AV synchrony is absent, the heart functions as an inefficient pump (largely because the right ventricle is contracting when it is not filled with blood).

According to a further aspect, the object of the invention is achieved by a method for vagal nerve stimulation, the method comprising:
- determining an exertion level indicating metabolic demand
- determining an autonomous status level, and
- delivering nerve stimulation pulses depending on both, the determined exertion level and the autonomic status level.

Preferably, nerve stimulation pulses having stimulation parameters that are configured to induce an increase in vagal tone are delivered if the cardiovascular demand representing signal of the activity sensor indicates a low demand and the autonomic status representing signal of the autonomic tone sensor indicates that the sympathetic tone is elevated.

It is further preferred if nerve stimulation pulses having stimulation parameters that are configured to induce a decrease in vagal tone and/or an increase in sympathetic tone are delivered, if the cardiovascular demand representing signal of the activity sensor indicates an elevated cardiovascular demand and the autonomic status representing signal of the autonomic tone sensor indicates that the sympathetic tone is suppressed.

The above and other aspects, features and advantages of the present invention will be more apparent from the following more particular description thereof, presented in conjunction with the following drawings wherein:
- Fig. 1: shows a vagal nerve stimulation system.
- Fig. 2: illustrates an implantable pulse generator for heart stimulation and vagus stimulation;
- Fig. 3: is a schematic block diagram of an implantable pulse generator (IPG) used in the system of Fig. 1.

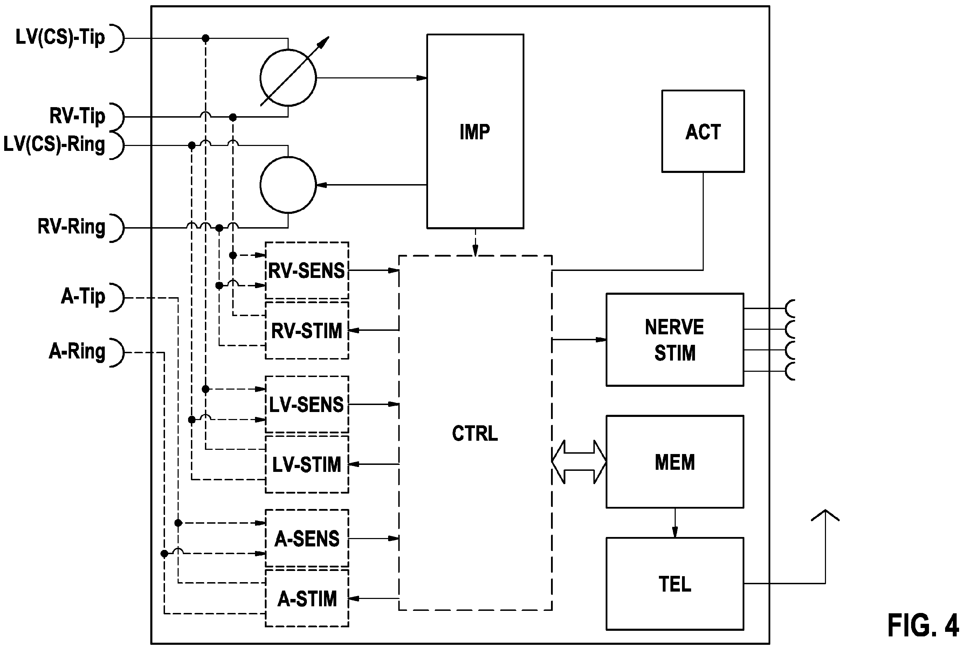

- Fig. 4: is a schematic block diagram of an implantable pulse generator (IPG) used in the system of Fig. 1 showing an alternative electrode arrangement.
- Fig. 5: is an illustration of one representative example of hysteresis control of VNS.
- Fig. 6: is an illustration of an another example of hysteresis control of VNS
- Fig. 7: illustrates an alternative means of hysteresis control of VNS.
- Fig. 8: is an illustration of adaptive delay for close loop control of VNS in response to transient change of patient's heart rate tone.

The following description is of the best mode presently contemplated for carrying out the invention. This description is not to be taken in a limiting sense, but is made merely for the purpose of describing the general principles of the invention. The scope of the invention should be determined with reference to the claims.

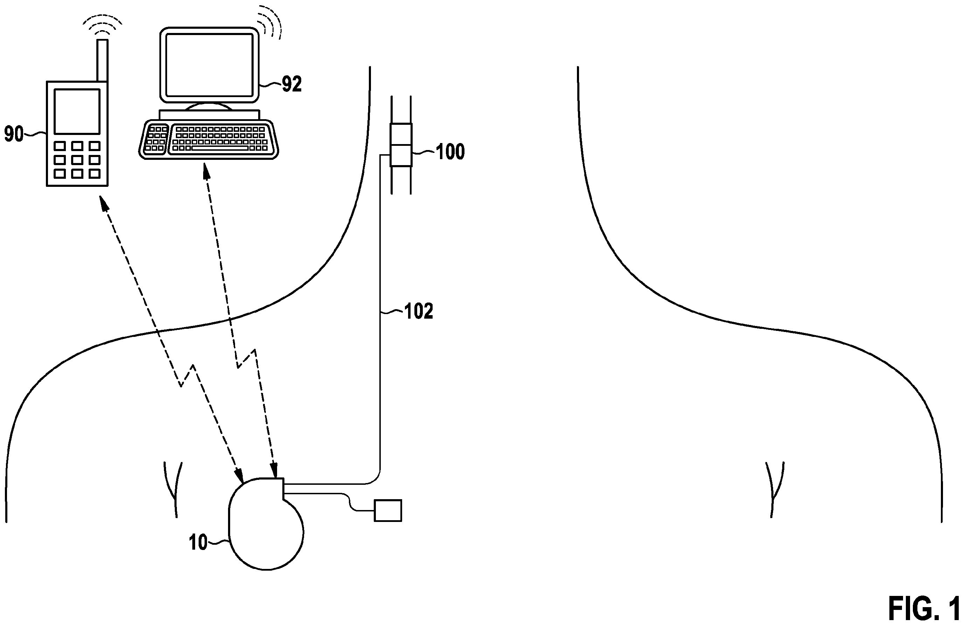

Fig. 1 illustrates an implantable pulse generator system for vagal nerve stimulation. The system comprises an implantable medical device 10 that is an implantable pulse generator (IPG). The implantable medical device 10 is connected to a nerve stimulation electrode cuff 100 via a nerve stimulation electrode lead 102. Additionally, nerve stimulation lead 102 can comprise one or more sensors for detecting cardiac signals, pressure signals or impedance signals.

The implantable pulse generator 10 can be wirelessly programmed by an external programmer 92 via a MICS-band link (or equivalent). The implantable pulse generator 10 can also communicate with a bedside Patient Messenger 90 via a similar link. Arrhythmia detection, blood pressure waveform changes, and the other relevant diagnostic parameters described before can be transmitted to the bedside Patient Messenger 90 who can alert a Home Monitoring/Remote Programming Center, if medical attention is required.

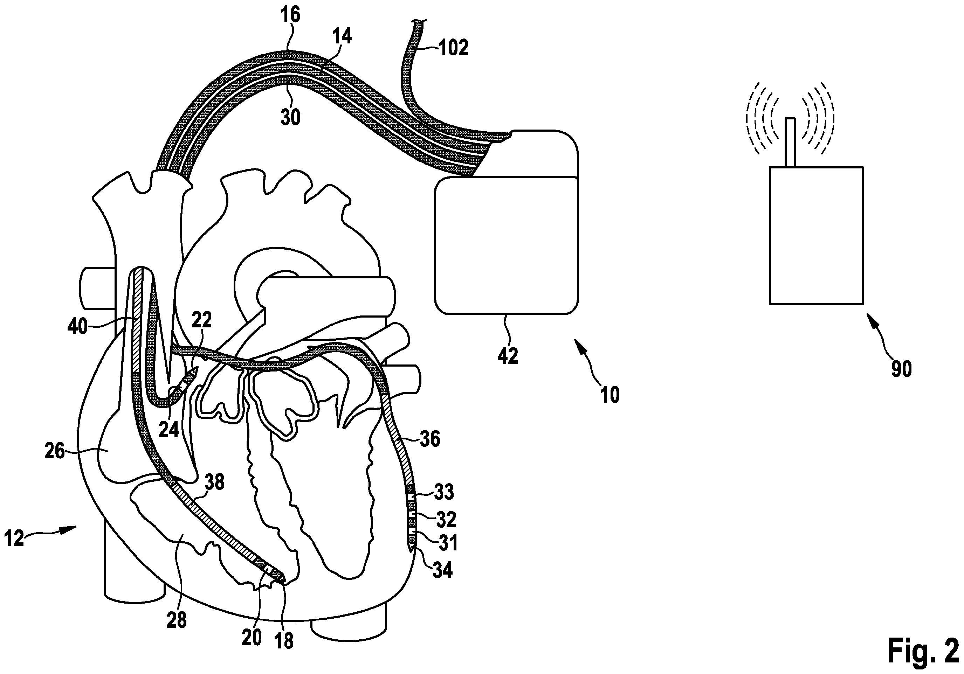

In figure 2 the implantable medical device (also referred to as implantable pulse generator) is a three chamber biventricular pacemaker and cardioverter/defibrillator that is connected to pacing/sensing leads placed in a heart 12. The implantable medical device 10 further is a vagal nerve stimulation pulse generator that is connected to cuff electrode 100 by means of nerve stimulation electrode lead 102.

As shown in figure 2, the preferred embodiment is to couple a vagal nerve stimulation pulse generator with an implantable bi-ventricular defibrillator.

The implantable medical device 10 is electrically coupled to heart 12 by way of leads 14, 16 and 30.

Lead 14 is a right atrial electrode lead that has a pair of right atrial electrodes 22 and 24 that are in contact with the right atria 26 of the heart 12.

Lead 16 is a right ventricular electrode lead that has a pair of ventricular stimulation and sensing electrodes 18 and 20 that are in contact with the right ventricle 28 of heart 12. Further, a ventricular defibrillation shock coil 38 and an atrial defibrillation shock coil 40 are arranged on lead 16.

Electrodes 22 and 18 are tip electrodes at the very distal end of leads 14 and 16, respectively. Electrode 22 is a right atrial tip electrode RA Tip and electrode 18 is a right ventricular tip electrode. Electrodes 24 and 20 are ring electrodes in close proximity but electrically isolated from the respective tip electrodes 22 and 18. Electrode 24 forms a right atrial ring electrode RA Ring and electrode 20 forms a right ventricular ring electrode RV Ring. Atrial cardioversion shock coil 40 is a coil electrode providing a relatively large geometric area when compared to the stimulation electrodes 18, 20, 22 and 24.

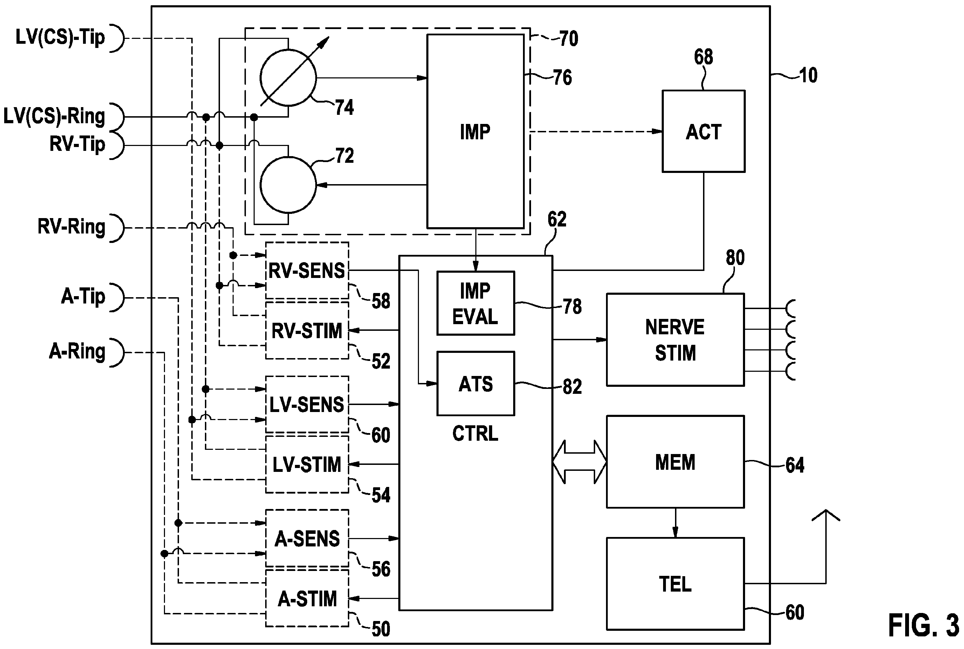

Lead 30 is a left ventricular electrode lead passing through the coronary sinus of heart 12 and having left ventricular ring electrodes LV RING 31, 32 and 33 and a left ventricular tip electrode LV TIP 34. Further, a left ventricular defibrillation shock coil 36 is arranged on lead 30. It is noted that the number of left ventricular ring electrodes may vary depending on the electrode lead that is used. In the context of figures 2 and 3, one left ventricular ring electrode LV-RING is referred to the acts as pars pro toto.

Implantable medical device 10 has a generator housing 42 made from electrically conductive material such as titanium that can serve as a large surface electrode IMD CASE.

The plurality of electrodes 18, 20, 22, 24, 31, 32, 33, 34, 36, 38 and 40 connected to implantable medical device 10 together with case 42 allow for a number of different electrode configurations for measuring intrathoracic and intracardiac impedance.

For each individual one of the intracardiac impedance measurements, injecting a forcing function from a right ventricular ring electrode to a left ventricular ring electrode and measuring a response function between the same electrodes (bipolar configuration; see figure 2) or, for instance, a right ventricular tip electrode and a left ventricular tip electrode (quadrupolar configuration; see figure 3) is possible. Further impedance measurement vectors (resulting from different impedance measurement electrode combinations) are possible.

Referring to figure 3 a simplified block diagram of an implantable medical device 10 is shown. During operation of the pacemaker leads 14, 16 and 30 are connected to respective output/input terminals of pacemaker 10 as indicated in figure 2 and carry stimulating pulses to the tip electrodes 18, 22 and 34 from a right atrial stimulation pulse generator A-STIM 50, a right ventricular pulse generator RV-STIM 52 and a left ventricular pulse generator LV-STIM 54, respectively. Further, electrical signals from the right atrium are carried from the electrode pair 22 and 24, through the lead 14, to the input terminal of a right atrial channel sensing stage A-SENS 56; and electrical signals from the right ventricle are carried from the electrode pair 18 and 20, through the lead 16, to the input terminal of a right ventricular sensing stage RV-SENS 58. Likewise electrical signals from the left ventricle are carried from the electrode pair 32 and 34, through the lead 30, to the input terminal of a left ventricular sensing stage LV-SENS 60.

Controlling the implantable medical device 10 is a control unit CTRL 62 that is connected to sensing stages A-SENS 56, RV-SENS 58 and LV-SENS 60 and to stimulation pulse generators A-STIM 50, RV-STIM 52 and LV-STIM 54. Control unit CTRL 62 receives the output signals from the atrial sensing stage A-SENS 56, from the right ventricular sensing stage RV-SENS 58 and from the left ventricular sensing stage LV-SENS 60. The output signals of sensing stages A-SENS 56, RV-SENS 58 and LV-SENS 60 are generated each time that a P-wave representing an intrinsic atrial event or an R-wave representing an intrinsic ventricular event, respectively, is sensed within the heart 12. An As-signal is generated, when the atrial sensing stage A-SENS 56 detects a P-wave and a RVs-signal is generated, when the right ventricular sensing stage RV-SENS 58 detects an R-wave.

These sense events are used by control unit CTRL 62 as fiducial points of the respective intracardiac electrograms picked up by the sensing stages A-SENS 56, RV-SENS 58 and LV-SENS 60, respectively.

Control unit CTRL 62 also generates trigger signals that are sent to the atrial stimulation pulse generator A-STIM 50, the right ventricular stimulation pulse generator RV-STIM 52 and the left ventricular stimulation pulse generator LV-STIM 54, respectively. These trigger signals are generated each time that a stimulation pulse is to be generated by the respective pulse generator A-STIM 50, RV-STIM 52 or LV-STIM 54. The atrial trigger signal is referred to simply as the "A-pulse", and the ventricular trigger signal is referred to as the "RV-pulse" or the "LV-pulse", respectively. During the time that either an atrial stimulation pulse or ventricular stimulation pulse is being delivered to the heart, the corresponding sensing stage, A-SENS 56, RV-SENS 58 and/or LV-SENS 60, is typically disabled by way of a blanking signal presented to these amplifiers from the control unit CTRL 62, respectively. This blanking action prevents the sensing stages A-SENS 56, RV-SENS 58 and LV-SENS 60 from becoming saturated from the relatively large stimulation pulses that are present at their input terminals during this time. This blanking action also helps prevent residual electrical signals present in the muscle tissue as a result of a stimulation pulse delivered from pacemaker 10 from being interpreted as P-waves or R-waves.

Furthermore, atrial sense events as recorded shortly after delivery of a ventricular stimulation pulses during a preset time interval called post ventricular atrial refractory period (PVARP) are generally recorded as atrial refractory sense event Ars but ignored.

Control unit CTRL 62 comprises circuitry for timing ventricular and/or atrial stimulation pulses according to an adequate stimulation rate that can be adapted to a patient's hemodynamic need as pointed out below.

Control unit CTRL 62 further comprises an autonomic tone sensor 82 that is connected to right ventricular sensing stage 58. The autonomic tone sensor 82 is configured to process IEGM signals representing an intracardiac electrogram (IEGM). The autonomic tone sensor 82 preferably is adapted to determine one or more of the following metrics from IEGM signals: intrinsic heart rate (HR), intrinsic atrioventricular (AV) conduction time, QRS duration and/or P wave duration.

Still referring to figure 3, the implantable medical device 10 includes a memory circuit MEM 64 that is coupled to the control unit CTRL 62 over a suitable data/address bus ADR. This memory circuit MEM 64 allows certain control parameters, used by the control unit CTRL 62 in controlling the operation of the implantable medical device 10, to be pro-grammably stored and modified, as required, in order to customize the implantable medical device's operation to suit the needs of a particular patient. Such data includes the basic timing intervals used during operation of the pacemaker 10 and AV delay values and hysteresis AV delay values in particular.

Further, data sensed during the operation of the implantable medical device 10 may be stored in the memory MEM 64 for later retrieval and analysis.

A telemetry circuit TEL 66 is further included in the implantable medical device 10. This telemetry circuit TEL 66 is connected to the control unit CTRL 62 by way of a suitable command/data bus. Telemetry circuit TEL 66 allows for wireless data exchange between the implantable medical device 10 and some remote programming or analyzing device which can be part of a centralized service center serving multiple pacemakers.

The implantable medical device 10 in figure 3 is referred to as a three chamber pacemaker/cardioverter/defibrillator because it interfaces with the right atrium 26, the right ventricle 28 and the left ventricle of the heart 12. Those portions of the pacemaker 10 that interface with the right atrium, e.g., the lead 14, the P-wave sensing stage A-SENSE 56, the atrial stimulation pulse generator A-STIM 50 and corresponding portions of the control unit CTRL 62, are commonly referred to as the atrial channel. Similarly, those portions of the pacemaker 10 that interface with the right ventricle 28, e.g., the lead 16, the R-wave sensing stage RV-SENSE 58, the ventricular stimulation pulse generator RV-STIM 52, and corresponding portions of the control unit CTRL 62, are commonly referred to as the ventricular channel.

In order to be able to determine a metabolic demand of a patient, the pacemaker 10 further includes a physiological sensor ACT 68 that is connected to the control unit CTRL 62 of the pacemaker 10. While this sensor ACT 68 is illustrated in figure 3 as being included within the pacemaker 10, it is to be understood that the sensor may also be external to the implantable medical device 10, yet still be implanted within or carried by the patient. A common type of activity sensor is an accelerometer, such as a piezoelectric crystal, mounted to the case of the pacemaker. Other types of physiologic sensors are also known, such as sensors that sense the oxygen content of blood, respiration rate, blood pH, intracardiac impedance changes, and the like. The type of activity sensor used is not critical to the present invention. Any sensor capable of sensing some physiological parameter relatable to physical activity and/or metabolic demand of a patient can be used. Such physiological sensors are commonly used with "rate-responsive" pacemakers in order to adjust the rate of the pacemaker in a manner that tracks the physiological needs of the patient. The output of sensor 68 represents an activity level.

According to a typical embodiment, the activity sensor 68 is an accelerometer. According to another embodiment, the activity sensor 68 is the CLS sensor, which has been shown to be able to measure both physical and mental exertion of the patient. Yet in another embodiment, the activity sensor 68 is the minute ventilation sensor, which may be based on the measurement of trans-thoracic impedance signal.

By means of the output signal of activity sensor 68 the control unit 62 is able to assign each intrinsic heart rate to an activity thus enabling collection of intrinsic heart rate value for a patient's state of rest and a patient's state of exercise separately.

For impedance measurement, an impedance determination unit 70 is provided. Impedance determination unit 70 comprises a constant current source 72 that is connected or can be connected to electrodes for intracorporeal placement as shown in figure 1. In order to allow for a plurality of impedance measurement electrode configurations, preferably some means of switching is provided between the constant current source 72 and the electrode terminals of the implantable medical device 10. The switch is not shown in figure 2. Rather, particular impedance measurement configurations are shown as examples.

Similarly, a voltage measuring unit 74 for measuring a voltage corresponding to a current fed through a body by said constant current source is provided and can be connected to a number of electrodes although a switch for switching between these configurations is not shown in figure 2.

As an alternative to constant current source 72 a constant voltage source can be provided to generate the forcing function. Then, the measuring unit will be adapted to measure a current strength of a current fed through a body by said constant voltage source.

Both, constant current source 72 and voltage measurement unit 74, are connected to an impedance value determination unit 76 that is adapted to determine an impedance value for each measuring current pulse delivered by the constant current source 72.

Further, an evaluation unit 78 is provided either as a separate unit or as part of control unit CTRL 62 as depicted in Figure 2. The evaluation unit 78 is connected to said impedance determination unit 70 and is adapted to evaluate a sequence of consecutive impedance values determined by said impedance determination unit 70. The evaluation unit 78 comprises a signal generator module (not shown) to construct the intracardiac impedance or conductance signal reflecting the time course of the impedance determination unit's output signal and its derivative.

The evaluation unit 78 further comprises a filter module (not shown) to filter the intracardiac impedance signal.

The evaluation unit 78 is further connected to the right ventricular stimulation stage RV-STIM 52 and the right ventricular sensing stage RV-SENS 58 in order to receive signals representing cardiac events, namely right ventricular stimulation events RVp or right ventricular sense events RVs, respectively.

The constant current source 72 has its two poles connected to different connectors for different electrodes as for example the right ventricular ring electrode and the left ventricular ring electrode (Fig. 2) or the left ventricular tip electrode and the right ventricular tip electrode (Fig. 3). The voltage measuring unit 74 has two poles connected to, for example, a connector for the left ventricular ring electrode and the right ventricular ring electrode (Fig. 2) or the left ventricular ring electrode and the right ventricular ring electrode (Fig. 3). Thus, a bipolar or a quadrupolar impedance measurement configuration is established.

Impedance measurement is carried out by injecting a constant current and sampling the resulting voltage.

The measuring current is preferably pulsed. Typically, the measuring current will feature biphasic pulses wherein two constant current pulses of opposite polarity form one pulse package. Two consecutive pulse packages between two consecutive pulse packages a time gap is provided, which is significantly longer than the duration of one pulse package. The constant current pulses within one pulse package are each of the same intensity and of same duration. They only have different polarities. The typical value for the intensity of the constant current pulses is between 50 uA and 600 µA. The typical pulse duration of a single constant current pulse is about 15 µs.

The time gap between each two consecutive pulse packages may be 500 times longer than the duration of one constant current pulse. The two constant current pulses of opposite polarity within one pulse package may not follow immediately each other but may have a time gap there between. This time gap however, will be very short compared to the time gap between two consecutive pulse packages. Furthermore, consecutive pulse packages may be face alternating such that a first pulse package for example will begin with a positive constant current pulse whereas the following pulse package will begin with a negative constant current pulse and end with a positive constant current pulse.

Via intracardiac impedance measurement, control unit 62 is able to determine a stroke volume.

Via intrathoracic impedance measurement control unit 62 is able to determine a tidal volume and a ventilation rate (breathing rate) in a manner generally known to the skilled person.

Implantable pulse generator 10 further comprises a nerve stimulation unit 80 for generating nerve stimulation pulses. The nerve stimulation unit 80 is connected to and controlled by control unit 62. When in use, nerve stimulation unit 80 is further connected to a nerve stimulation electrode lead which preferably is a quadrupolar lead comprising four electric conductors the can connect the nerve stimulation unit 80 to corresponding stimulation electrode poles of a nerve stimulation electrode cuff.

Hysteresis is an intrinsic behavior of cardiovascular system, respiratory system, and many other physiological systems. It regulates the normal function of human body based on not only the current physiological status, but also its past history.

VNS is a known therapy to treat many diseases, including heart failure. However, over treatment by VNS may be associated with the risk of severe sympathetic suppression, leading to side effects such as bradycardia. Simply turning on/off the VNS therapy based on a predefined heart rate cutoff value is not optimal because it does not consider adapting VNS to the past history of patient's physiological status. Introducing hysteresis control to VNS may ensure more physiological adaptation of the VNS therapy to the changing autonomic tone.

According to this invention, the VNS system comprises an autonomic tone sensing unit which measures a physiological signal that is known to be affected by the autonomic status. For example, the system can use IEGM sensing electrodes to measure the intrinsic heart rate (HR) which is directly modulated by the autonomic status of the patient. Elevated sympathetic tone leads to increase in HR and vice versa. In another example, the intrinsic AV conduction time is known to be affected by the autonomic status of the patient. Elevated sympathetic tone is associated with decrease in intrinsic AV conduction time and vice versa. Other physiological parameters can also be measured for the evaluation of autonomic status, including but are not limited to, QRS duration, P wave duration, heart sound, intracardiac pressure, minute ventilation, etc. In the following description, the HR is used as an example of autonomic tone indicator to illustrate the concept of the invention, while it should be understood that other metrics can also be used to measure patient's autonomic tone.

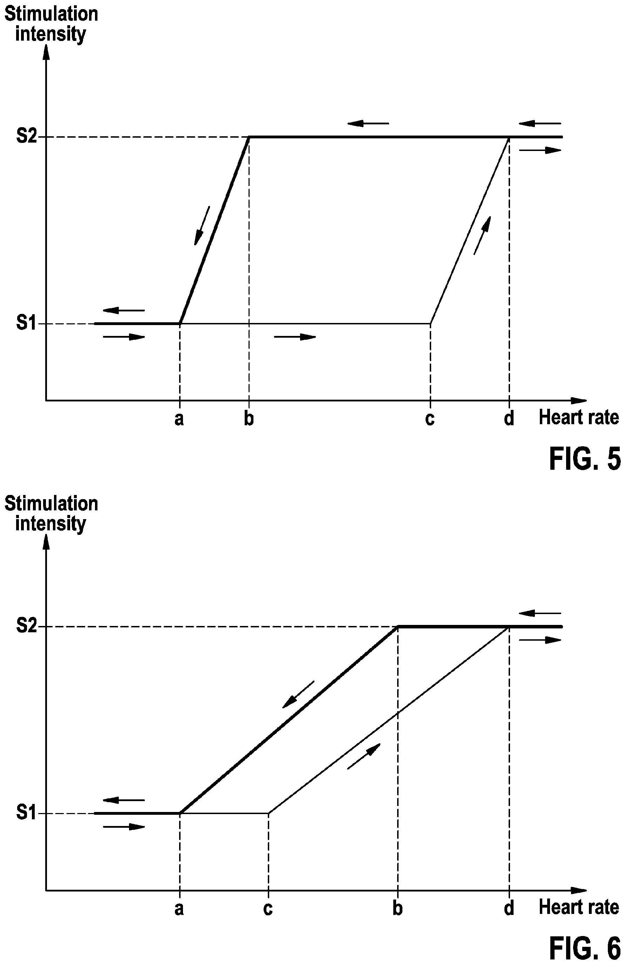

According to one embodiment of this invention, hysteresis is used to control VNS. Generally, within a predetermined range of autonomic tone (i.e. a predefined HR range), the intensity of VNS gradually increases when the sensed autonomic tone is shifting toward more sympathetic dominance (e.g. increase of HR), and gradually decreases when the sensed autonomic tone is shifting toward more parasympathetic dominance (e.g. decrease of HR). The increase and decrease of VNS intensity follow two different curves. Specifically, at a specific autonomic tone within the predetermined range (e.g. a specific HR within the predefined HR range), the VNS intensity is higher if autonomic tone is shifting toward more parasympathetic dominance (e.g. decrease of HR) than the VNS intensity if autonomic tone is shifting toward more sympathetic dominance (e.g. increase of HR). Figures 5, 6, and 7 illustrate three different examples of hysteresis control of VNS.

In Figure 5, heart rates 'a', 'b', 'c' and 'd' define the hysteresis curves, where a<b<c<d, and these parameters are predefined or user-programmable. As HR increases, the intensity of vagal nerve stimulation does not increase until HR is higher than 'c' and reaches full intensity S2 when HR reaches 'd'. As heart rate decreases, the intensity of VNS does not decrease until HR is lower than b and reaches lowest intensity S1 when HR reaches 'a'.

The example shown in Figure 6 is similar to that in Figure 1. Similarly, heart rates 'a', 'b', 'c' and 'd' define the hysteresis curves, but HR b is greater than HR c (note: in another example not shown here, heart rates b and c are equal, i.e. b=c). Likewise, these heart rates are predefined or user-programmable. As HR increases, the intensity of VNS does not increase until HR is higher than 'c' and reaches full intensity S2 when HR reaches 'd'. As heart rate decreases, the intensity of vagal nerve stimulation does not decrease until HR is lower than b and reaches lowest intensity S1 when HR reaches 'a'.

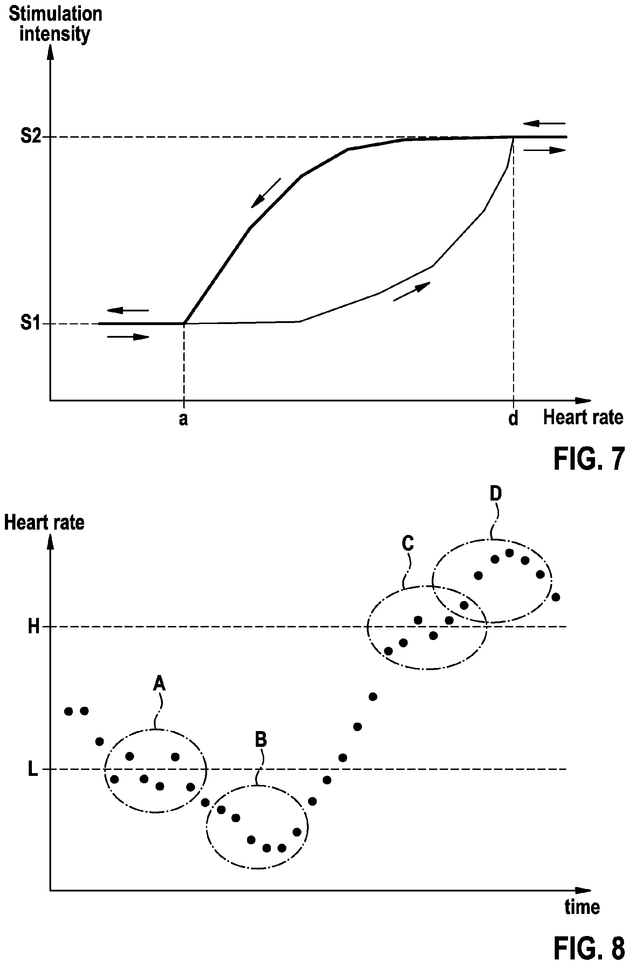

In the example shown in Figure 7, two heart rates 'a' and 'd' are predefined or user programmable, and a<d. The VNS keeps at the lowest intensity S1 when HR <= a. It gradually increases toward S2 when HR is greater than 'a' but is less than 'd'. Then when HR >= d, the VNS reaches the highest intensity S2.

In above examples, S1 and S2 are preferably user-programmable VNS parameters. As known in the art, the VNS intensity (incl. S1 and S2) can be controlled by programming stimulation amplitude, pulse width, pulse frequency, and/or other pulse timing such as duty cycling. In one special embodiment, S1 is defined as VNS off.

According to another embodiment of this invention, modulation of VNS intensity is delayed in an adaptive manner in response to the transient change of patient's autonomic tone. As described above, patient's autonomic tone can be measured by a plurality of means. To illustrate the concept, patient's heart rate is used as an indicator of patient's autonomic tone in the following descriptions, while it should be understood that other metrics can also be used to measure patient's autonomic tone.

As illustrated in Figure 8, adaptive delay of VNS intensity modulation is introduced as a means of close loop control of VNS in response to transient change of patient's heart rate (as an indicator of autonomic tone). In this figure, each small circle represents an instantaneous heart rate after one cardiac cycle. The VNS device continuously monitors the heart rate and evaluates patient's autonomic tone based on the measured heart rate. The device also evaluates the severity of sympathetic elevation when the heart rate is persistently high and the severity of vagal depression when the heart rate is persistently low. Based on such evaluation, the device further modulates the intensity of VNS therapy. Transient change of heart rate measured by the device, for example, due to natural heart rate variability or the measurement noise, is managed by means of an adaptive delay function as described in details below, to prevent the device from frequently and unnecessarily changing the intensity of VNS therapy.

In preferred embodiment, at least one high heart rate limit and one low rate limit are predefined. In the example shown in Figure 8, a high heart rate limit (H) and a low heart rate limit (L) are predefined. The device continuously measures the instantaneous heart rate and evaluates the severity of sympathetic elevation and the severity of vagal suppression in a moving window (in the example shown in Figure 8, the size of the moving window is set to 6 cardiac cycles). In one exemplary embodiment, the severity of sympathetic elevation is quantitatively calculated as SSE = ∑[R(i)-H], and the severity of vagal depression is quantitatively calculated as SVD = ∑[L-R(i)], where i is the beat index, R(i) is the instantaneous heart rate at the i-th beat, and the summation is performed over all beats in the moving window. Therefore, a beat with instantaneous heart rate dropping below L will increase SVD, and a beat's heart rate higher than L will decrease SVD. Similarly, a beat with instantaneous heart rate higher than H will increase SSE, while a beat's heart rate lower than H will decrease SSE.

Preferably, when the calculated SSE exceeds a predefined sympathetic tone threshold (STH), VNS is modulated upward to increase the intensity of stimulation. On the other hand, when the calculated SVD exceeds a predefined vagal tone threshold (VTH), VNS is modulated downward to decrease the intensity of stimulation. Because the calculation of SSE and SVD is performed over a moving window, transient decrease of heart rate below L will be balanced by those beats above L (see 'A' in Figure 8), thus will not trigger modulation of VNS therapy. Only when the heart rate is persistently below the target rate L such that the calculated SVD exceeds the predefined VTH (see 'B' in Figure 8), downward modulation of VNS is initiated. Similarly, transient increase of heart rate above H will be balanced by those beats below H (see 'C' in Figure 8), thus will not trigger modulation of VNS therapy. Only when the heart rate is persistently above the target rate H such that the calculated SSE exceeds the predefined STH (see 'D' in Figure 8), upward modulation of VNS is activated.

It should be understood that the calculation of SSE and SVD as the equation shown above is merely for the purpose of illustrating the concept. Other formulas can be developed to quantify SSE and SVD. For example, a set of predefined coefficients can be applied to the difference [R(i)-H] for calculation of SSE, while another set of predefined coefficients can be applied to the difference between [L-R(i)], and these coefficients can be rate dependent as well. In another example, the difference terms [R(i)-H] and [L-R(i)] can be scaled nonlinearly. Yet in another example, the difference terms [R(i)-H] and [L-R(i)] can be respectively replaced by the evaluating the differences in duration of cardiac intervals. For example, SSE can be instead evaluated by ∑[60/H - 60/R(i)], while SVD can be instead evaluated by ∑[60/R(i) - 60/L]. Of course, the corresponding sympathetic tone threshold (STH) and vagal tone threshold (VTH) should be changed to have the corresponding unit of time duration.

Although Figure 8 illustrates the concept of adaptive delay of VNS modulation using two heart rate limits (H and L), it should be understood that more than one high rate limits (e.g. H1 and H2) and/or more than one low rate limits (e.g. L1 and L2) can be predefined to achieve graded control of VNS. For example, the severity of sympathetic elevation (SSE1 and SSE2) can be calculated respectively for two different high rate threshold values (H1 and H2) and compared with two different sympathetic tone thresholds (STH1 and STH2). Correspondingly, the degree of upward modulation of VNS can also be different when SSE1 exceeds STH1, compared to when SSE2 exceeds STH2. Similarly, the severity of vagal depression (SVD1 and SVD2) can be calculated respectively for two different low rate threshold values (L1 and L2) and compared with two different vagal tone thresholds (VTH1 and VTH2). Correspondingly, the degree of downward modulation of VNS can also be different when SVD1 exceeds VTH1, compared to when SVD2 exceeds VTH2.

### LIST OF REFERENCES:

- 10: implantable pulse generator
- 12: heart
- 14, 16, 30: leads
- 18, 20: pair of ventricular stimulation and sensing electrodes
- 22, 24: right atrial electrodes
- 26: right atria
- 28: right ventricle
- 31,32,33: left ventricular ring electrodes (LV RING)
- 34: left ventricular tip electrode (LV TIP)
- 36: left ventricular defibrillation shock coil
- 38: ventricular defibrillation shock coil
- 40: atrial defibrillation shock coil
- 42: case
- 50: right atrial stimulation pulse generator (A-STIM)
- 52: right ventricular pulse generator (RV-STIM)
- 54: left ventricular pulse generator (LV-STIM)
- 56: right atrial channel sensing stage (A-SENS)
- 58: right ventricular sensing stage (RV-SENS)
- 60: left ventricular sensing stage (LV-SENS)
- 62: control unit (CTRL)
- 64: memory circuit (MEM)
- 66: telemetry circuit (TEL)
- 68: activity sensor (ACT)
- 70: impedance determination unit
- 72: current source
- 74: voltage measuring unit
- 76: impedance value determination unit (IMP)
- 78: evaluation unit (IMP-EVAL)
- 80: nerve stimulation unit (NERVE-STIM)
- 82: autonomic tone sensor (ATS)
- 90: Patient Messenger
- 92: external programmer
- 100: cuff electrode
- 102: nerve stimulation electrode lead

## Claims

1. Implantable pulse generator system (10) comprising
a nerve stimulation unit (80) for generating and delivery of vagal nerve stimulation pulses,
at least one autonomic tone sensor (82) for determining an autonomic status of a user and generating an autonomic status representing signal and
a control unit (62) that that is connected to the nerve stimulation unit and the autonomic tone sensor (82) and that is configured to control the nerve stimulation unit (80) to generate vagal nerve stimulation pulse trains that are adapted to cause vagal nerve stimulation (VNS) with varying intensity, depending on the autonomic status representing signal,
wherein the control unit (62) is further configured to evaluate the autonomic status representing signal in a moving window and to thus generate an evaluated autonomic status representing signal and
wherein the control unit (62) is further configured to gradually increase the intensity of VNS when the evaluated autonomic status representing signal indicates an autonomic tone that is shifting toward more sympathetic dominance, and gradually decrease the intensity of VNS when the autonomic status representing signal indicates an autonomic tone that is shifting toward more parasympathetic dominance, wherein the gradual increase and the gradual decrease of the intensity of the VNS follow two different curves.

2. Implantable pulse generator system (10) according to claim 1, wherein the autonomic status representing signal represents the instantaneous heart rate on a beat-to-beat or the instantaneous atrio-ventricular conduction time and wherein the moving window includes a predetermined number of heart rate values or atrio-ventricular conduction time values, respectively.

3. Implantable pulse generator system (10) according to claim 2, wherein the control unit (62) is further configured to generate the evaluated autonomic status representing signal based on the average of the heart rate values or the atrio-ventricular conduction time values, respectively, in the moving window.

4. Implantable pulse generator system (10) according to at least one of claims 1 to 3, wherein the control unit (62) is further configured to increase the intensity of the VNS in response to a decrease of the heart rate or an increase of the atrio-ventricular conduction time and to decrease the intensity of the VNS in response to an increase of the heart rate or a decrease of the atrio-ventricular conduction time.

5. Implantable pulse generator system (10) according to at least one of claims 1 to 4, wherein the control unit (62) is further configured to cause an increase or a decrease of the intensity of the VNS only when the autonomic status representing signal or the evaluated autonomic status representing signal is within a predetermined range of values.

6. Implantable pulse generator system (10) according to at least one of claims 1 to 5, wherein at least one high heart rate limit (H) and one low heart rate limit (L) are predefined and the implantable pulse generator system is configured to continuously determine the instantaneous heart rate and the control unit is configured to evaluate the severity of sympathetic elevation (SSE) and the severity of vagal suppression (SVD) in a moving window.

7. Implantable pulse generator system (10) according to claim 6, wherein the control unit (62) is configured to determine the severity of sympathetic elevation as SSE = ∑[R(i)-H], and the severity of vagal depression as SVD = ∑[L-R(i)], where i is the beat index, R(i) is the instantaneous heart rate at the i-th beat, and the summation is performed over all beats in the moving window.

8. Implantable pulse generator system (10) according to claim 7, wherein the control unit (62) is configured to increase the intensity of VNS when the calculated SSE exceeds a predefined sympathetic tone threshold (STH) and to decrease the intensity of VNS when the calculated SVD exceeds a predefined vagal tone threshold (VTH), VNS is modulated downward to decrease the intensity of stimulation.

9. Implantable pulse generator system (10) according to at least one of claims 6 to 8, wherein the control unit (62) is configured to apply a first set of predefined coefficients to the difference [R(i)-H] for calculation of SSE, and to apply a second set of predefined coefficients to the difference between [L-R(i)].

10. Implantable pulse generator system (10) according to claim 9, wherein the control unit (62) is configured to determine the coefficients depending on an instantaneous heart rate.

11. The implantable pulse generator system (10) according to at least one of claims 1 to 10, wherein the control unit (62) is adapted to control the nerve stimulation unit (80) to generate stimulation pulses having stimulation parameters that are configured to induce a VNS effect that is either an increase in vagal tone or an increase in sympathetic tone, depending on both, the cardiovascular demand representing signal and the autonomic status representing signal.

12. The implantable pulse generator system (10) according to at least one of claims 1 to 11, wherein the control unit (62) and the nerve stimulation unit (80) are adapted to increase or decrease the intensity of VNS by adjusting stimulation parameters including at least one of amplitude, pulse width and/or pulse frequency.

13. The implantable pulse generator system (10) according to at least one of claims 1 to 12, wherein the control unit (62) is connected to the activity sensor and the autonomic tone sensor (82) and is configured to control the stimulation unit depending on both, the metabolic demand-representing signal and the autonomic status-representing signal.

14. The implantable pulse generator system (10) according to claim 13, wherein the activity sensor (68) comprises at least one of an accelerometer, a CLS sensor and/or a minute ventilation sensor.
